# EUROPEAN PATENT SPECIFICATION

(11) **EP 1 381 429 B1**
(45) Date of publication and mention of the grant of the patent: **14.02.2007**
(21) Application number: 01274058.5
(22) Date of filing: 30.11.2001
(51) Int. Cl.: A61N 5/06

(54) **THERAPEUTIC TREATMENT DEVICE**
THERAPEUTISCHE BEHANDLUNGSVORRICHTUNG
DISPOSITIF ET METHODE DE TRAITEMENT D'AFFECTIONS CUTANEES ET D'ELIMINATION DE POILS INDESIRABLES PAR ECLAIRAGE DE LA PEAU AU MOYEN D'UNE LUMIERE INCOHERENTE

(30) Priority: 30.03.2001 DK 200100550
(43) Date of publication of application: 21.01.2004
(73) Proprietor: Cyden Ltd., Swansea SA1 8PH (GB)
(72) Inventor: SIMONSEN, Jan, Henning, DK-7600 Struer (DK); BJERRING, Peter, DK-8240 Risskov (DK)
(74) Representative: Austin, Hedley William
(86) International application number: PCT/DK2001/000799
(87) International publication number: WO 2002/078786

(56) References cited:
- EP-A2- 0 565 331
- EP-A2- 0 736 308
- SE-B- 465 953
- US-A- 5 989 283
- DATABASE WPI Week 200161, Derwent Publications Ltd., London, GB; AN 01-544766, XP002971278 & JP 2001 197921 A (DREA-N, DREAM KK) 24 July 2001

## Description

The present invention relates to a therapeutic treatment device especially for treatment of skin disorders such as removal of unwanted hair, discolouring, smoothening or wrinkles.

### Background of the invention

It is known in the prior art to use electromagnetic radiation in medical application for therapeutic uses such as treatment of skin disorders. For example, US patent No. 4,298,005 to Mutzhas describes a continuous ultraviolet lamp with cosmetic, photobiological, and photochemical applications. A treatment based on using the UV portion of the spectrum and its photochemical interaction with the skin is described. The power delivered to the skin using Mutzhas' lamp is described as 150 W/m.sup 2, which does not have a significant effect on skin temperature.

In addition to prior art treatment involving UV light, lasers have been used for dermatological procedures, including Argon lasers, CO₂ lasers, Nd(Yag) lasers, Cooper vapour lasers, ruby lasers and dye lasers. For example, US patent No. 4,829,262 to Furumoto, describes a method of constructing a dye laser used in dermatology applications. Two skin conditions which may be treated by laser radiation are external skin irregularities such as local differences in the pigmentation or structure of the skin, and vascular disorders lying deeper under the skin which cause a variety of skin abnormalities including port wine stains, telangiectasias, leg veins and cherry and spider angiomas. Laser treatment of these skin disorders generally includes localised heating of the treatment area by absorption of laser radiation. Heating the skin changes or corrects the skin disorder and causes the full or partial disappearance of the skin abnormality.

Certain external disorders such as pigmented lesions can also be treated by heating the skin very fast to a high enough temperature in order to evaporate parts of the skin.

Deeper-lying vascular disorders are more typically treated by heating the blood to a high enough temperature to cause it to coagulate. The disorder will then eventually disappear. To control the treatment depth a pulsed radiation source is often used. The depth the heat penetrates in the blood vessel is controlled by controlling the pulse width of the radiation source. The absorption and scattering coefficients of the skin also affect the heat penetration. These coefficients are a function of the constituents of skin and the wavelength of the radiation. Specifically, the absorption coefficient of light in the epidermis and dermis tends to be a slowly varying, monotonically decreasing function of wavelength. Thus, the wavelength of the light should be condition and vessel size being treated.

The effectiveness of lasers for applications such as tattoo removal and removal of birth and age marks is diminished because laser are monochromatic. A laser of a given wavelength may be effectively used to treat a first type of skin pigmentation disorder, but, if the specific wavelength of the laser is not absorbed efficiently by skin having a second type of disorder, it will be ineffective for the second type of skin disorder. Also, lasers are usually complicated, expensive to manufacture, large in comparison to the amount of power delivered, unreliable and difficult to maintain.

The wavelength of the light also affects vascular disorder treatment because blood content in the vicinity of the vascular disorders varies, and blood content affects the absorption coefficient of the treatment area. Oxyhemoglobin is the main chromophore which controls the optical properties of blood and has strong absorption bands in the visible region. More particularly, the strongest absorption peak of oxyhemoglobin occurs at 418 nm and has a band-width of 60 nm. Two additional absorption peaks with lower absorption coefficients occur at 542 and 577 nm. The total band-width of these two peaks is on the order of 100 nm. Additionally, light in the wavelength range of 500 to 550 nm is desirable for the treatment of blood vessel disorders of the skin since it is absorbed by the blood and penetrates through the skin. Longer wavelengths up to 1000 nm are also effective since they can penetrate deeper into the skin, heating the blood vessel by thermal conductivity. Also, longer wavelengths are effective for treatment of larger diameter vessels because the lower absorption coefficient is compensated for by the longer path of light in the vessel.

Accordingly, a wide band electromagnetic radiation source that covers the near UV and the visible portion of the spectrum would be desirable for treatment of external skin and vascular disorders. The overall range of wavelengths of the light source should be sufficient to optimise treatment for any of a number applications. Such a therapeutic electromagnetic radiation device should also be capable of providing an optimal wavelength range within the overall range for the specific disorder being treated. The intensity of the light should be sufficient to cause the required external thermal effect by raising the temperature of the treatment area to the required temperature. Also, the pulse-width should be variable over a wide enough range so as to achieve the optimal penetration depth for each application.

Therefore, it is desirable to provide a light source having a wide range of wavelengths, which can be selected according to the required skin treatment, with a controlled pulse-width and a high enough energy density for application to the affected area.

Pulsed non-laser type light sources such as linear flash lamps or flash bulbs provide these benefits. The intensity of the emitted light can be made high enough to achieve the required thermal effects. The pulse-width can be varied over a wide range so that control of thermal depth penetration can be accomplished. The typical spectrum covers the visible and ultraviolet range and the optical bands most effective for specific applications can be selected, or enhanced using fluorescent materials.

Moreover, non-laser type light sources such as flash bulbs are much more simple and easier to manufacture than lasers, are significantly less expensive for the same output power and have the potential of being more efficient and more reliable. They have a wide spectral range that can be optimised for a variety of specific skin treatment applications. These sources also have a pulse length that can be varied over a wide range which is critical for the different types of skin treatments.

Furthermore, another problem exists with the laser type devices known for these kinds of treatments. First of all the larger devices are very expensive to manufacture which only makes them available for hospitals or large clinics, which have a lot of patients or have patients who are capable of paying for the very expensive treatment.

In this connection the treatment is carried out by specially trained personnel since the laser devices are very powerful light emitting devices. It takes skill and training to be able to adjust the power of the laser and the spectrum of the laser light such that the optimal treatment will be performed on the patients. The amount of energy which the laser device should transmit to the patient's skin is dependent on many factors of the patient skin type, skin moisture, skin colouring, what type of treatment should be performed etc.

If the person carrying out the treatment does not posses the necessary skills the operator might perform a bad treatment. By using a tool with a high power intensity on the laser, the skin will discolour and furthermore, laser treatments are as mentioned above monochromatic which means that there is only one wavelength of light being pulsed at the treatment area.

Furthermore, the known machines only provides for one short but intense light pulse. In some treatments one short but powerful impulse can generate a lot of heat in the skin and thereby cause pain to the patient but also severe burns or discolouring on the skin of the patient.

Documents EP-A-0 565 331, EP-A-0 736 308, USA-A-5 989 283 and WO-A-91/15 264 disclose therapy treatment devices according to the preamble of claim 1.

There is therefore, a need to develop a device which can carry out a therapeutic treatment of the types mentioned above but which to the patient is cheap, easy and safe to operate and preferably can be operated by the patient himself, which will make it more readily available to the broad public and thereby not reserve this kind of treatments for the few who can afford the expensive treatments in hospitals.

The present invention solves this problem by providing a device according to claim 1.

The present invention has been developed in order to be able to carry out therapeutic treatment of all the types listed above. The invention is especially useful for removing unwanted hair and to smoothen out wrinkles. By providing a treatment device which comprises a disposable incoherent light source the used/spent disposable light source is replaced by another disposable light source, for example of a different category. Hereby, is facilitated that light sources with different characteristics for different treatments can be used with the same inexpensive device.

During the development of the present device it was realised that especially for removal of unwanted hair growth very few hair light pulses were needed at each treatment session. On the other hand when the treatment was to smoothen out wrinkles, light of a different wavelength as well as a lower energy level was desired and more pulses were shown to be useful. It is therefore desirable to be able to change the characteristics of the disposable light source according to the desired treatment.

The difference in number of light pulses as well as intensity of light pulses shall be seen in the effect that light energy has on the skin respectively the hair follicle.

By being able to direct light at a certain intensity at the skin this will stimulate the production of collagen which keeps the skin smooth and elastic and thereby improves and smoothens out the wrinkles in the skin.

On the other hand when wanting to remove unwanted hair and avoid the immediate regrowth of hair, it is desirable to use a higher intensity in order to burn away the follicle. This can most advantageously be done by first creating a light pulse which will preheat the area and then after a certain period follow the preheating with the actual destroying light pulse.

Since the present invention is able to carry out the therapeutic treatment with respect to the different treatments as mentioned above with a rather low energy density the risk of creating discolouring, burns or other unwanted side effects is minimised.

With the devices treating the skin with laser light it is difficult to determine the exact amount of laser power which should be used to treat the single patient as there are many factors relating to the skin of the patient which has effect on the success of treatment. Especially, in this connection the skin's content of melanin which absorbs and distributes light within the skin is very important.

From the above it should be clear that with the present invention it is important to provide disposable light sources which can give out one or more light pulses at relatively low energy levels within the desired wavelength band widths. This is done according to the invention by providing a disposable light source which comprises one or more ignitable materials, and wherein the layers are separated by a delaying fuse such that when the disposable light source is ignited or triggered a first layer will create a first light pulse, once this light pulse has burned out the delaying fuse will transmit the trigger ignition with a certain delay to the second layer, which will thereafter ignite and create another light pulse and after this light pulse has died the delaying fuse layer will delay the ignition of a third layer etc. etc. In this way a disposable light source having the capability of emitting one or more light pulses is created.

Furthermore, the intensity as well as the duration of the light pulse can be varied by varying the trigger current. As well as arranging more ignition devices, i.e. electrodes which can ignite different or separate parts of the ignitable material.

In an alternative embodiment the ignitable materials can be in the shape of one or more metal meshes which are arranged with separate electrodes such that they or each mesh can_be ignited separately by a triggering device such that one mesh can ignite followed by the next mesh and thereby create a series of light pulses.

The emitted light will be so-called white light but depending on the materials which are used as ignitable materials, the wavelength of the emitted light can be somewhat controlled. Furthermore, by selecting an appropriate amount of material as well as appropriate additives to the materials, the length of the light pulse can be varied. For the therapeutic treatment according to the invention it has been found that light pulses between 15 millisec. and up to 2 sec. are preferable.

By placing the meshes as described above in a controlled atmosphere a further refined band of wavelength can be obtained. The controlled atmosphere can for example be Xenon or Oxygene.

Research with the present invention has shown that disposable flash bulbs in a modified form are advantageously used in devices according to the invention. The traditional disposable flash bulbs can be designed to have the required light pulse length as mentioned above from 15 millisec. to about 2 sec., whereas an electronic flash is much faster and not useful for the purposes within the scope of the present invention.

As traditional flash light bulbs are comparable to normal light bulbs in that the light is emitted in an even distribution to the surroundings, it can for the purpose of the present invention be advantageous to collect the emitted light and guide it towards the area which is to be treated. This can in an advantageous embodiment of the invention be done by arranging a prism between the light source and the object to be treated such that the prism device collects and guides the emitted light to the object to be treated.

By providing such a prism in order to collect and thereby concentrate the light emitted from the disposable light source on to the surface of the treatment area, it is possible to carry out the treatment with a lower energy consumption as more light is collected and transmitted on to the treatment zone than what could be expected, if the light was just transmitted freely to the surroundings.

In a further advantageous embodiment the prism device consists of a plurality of smaller separate prisms arranged to collect and concentrate and guide the emitted light towards a treatment surface. By splitting the prism up into a number of separate prisms it will be possible to distribute the emitted light more evenly on the surface to be treated and thereby create a more controlled and even energy level transmitted to the treatment surface than if the light was just freely emitted and transmitted to the treatment surface. Furthermore, by evenly collecting and distributing the light, it will be possible to treat larger surfaces pr. pulse.

In a further advantageous embodiment of the invention the prism has two more important uses. One of these is to keep the light source at a fixed distance from the treatment surface. By giving the prism a certain thickness it is possible to assure that there is a certain distance between the surface to be treated and the light emitting source.

In practical use this is done by having a disposable light source with a rather extended prism which is adapted to be touching the skin during treatment. Hereby the prism acts as a kind of distance keeper in order to be able to control the precise energy delivery to the treatment area.

The second function of this prism device is as a safety measure. By making the disposable light source stronger in the direction which is supposed to be in contact with the treatment area, it is less likely that the disposable light source will fracture in the lens and thereby hurt the patient who is being treated. This is important as the traditional flash bulbs are usually made of rather thin glass and by having a material placed within the bulb which is ignited and thereby explodes, it can fracture and shatter due to faults in the glass. The ignitable material can cause explosions which are too powerful for the rather thin glass to withstand which then can result in fractured glass.

In yet another further advantageous embodiment of the invention the sides of the prism are coated with a laser dye. Laser dyes are used to transform light at one wavelength into light at another wavelength. By selecting the ignitable material to ignite and emit light at an appropriate wavelength, this can further be refined by applying a laser dye, which will further limit the band of wavelength which is emitted through the prism as such. It is hereby possible to further improve the controlled energy transmission of light from the disposable light source to the treatment area on the patient. This is important as described above in that it hereby is possible to carry out successful treatments at lower energy levels.

In a further advantageous embodiment a concave reflecting element is arranged proximate to the light source such that it can reflect the light in a general direction towards the treatment area.

By arranging a reflector around the light source similar advantages as arranging a prism in front of the light source is achieved, namely, that the light is collected and guided in a general direction towards to treatment surface. By this arrangement more of the emitted light energy is utilised and thereby it is possible to operate the device at lower energy levels, which again makes the device safer to use.

In a further embodiment the device is both supplied with a reflecting device arranged in a housing around the light source as well as an iris or prism in front of the light source, wherein the iris or prism spans the opening in the housing through which the light is emitted towards the treatment surface such that the light emitted from the disposable light source is reflected from the reflector through the prism and on to the treatment surface. In this manner substantially all the light emitted from the disposable light source is utilised in the treatment of the skin or hair.

In an especially advantageous embodiment the reflecting device is made with a plurality of concave surface indentations. These indentations serve to collect and distribute the emitted light. By arranging a multitude of these indentations evenly on the reflector the resulting emitted light from the device will be of a more even character as the multitude of indentations will diffuse the light evenly over the opening in the device.

In another advantageous embodiment the device is constructed as a self contained unit, which can be compared to a well known flash light device in that an energy source in the shape of one or more batteries is supplied and connected to the disposable incoherent light source in the form of a flash bulb and that in between the energy source and the flash bulb is arranged a trigger device. This trigger device can be in the shape of a switch which is an on/off mechanism such that either current is supplied to the flash device or it is not supplied.

In other embodiments of the invention especially where flash bulbs containing metal meshes which have separate sets of electrodes the triggering device can be in the shape of a circuit, which will when the device is activated, trigger the sets of electrodes according to a pre-set routine with pre-set intervals between each ignition of a set of electrodes.

In more advanced embodiments of the invention sensor means can be arranged at the front end of the device in close proximity to the treatment area. These sensor means can detect the type of skin, the colour of the skin, the reflection characteristics of the skin etc. and through data computing collecting and processing means control the ignition of the disposable light source such that an optimum treatment is carried out in accordance with the skin type to be treated. Alternatively, the device can comprise means to indicate the most suitable light source in relation to the data collected by the sensor means.

This is a very advantageous embodiment in that the device itself takes into account the individual users special skin types and requirements which therefore will assure an optimum treatment according to the circumstances.

In a further development of the sensor means the sensor can be made to recognise, whether or not the treatment device is in contact with the treatment area of skin or is by mistake placed for example in too close proximity to an eye or other organ which can be damaged by intense light. In this connection the sensor means is acting as sort of a safety measure in order to make sure that the treatment device is only used on skin areas of the body. The sensor means, safety means then works by blocking the trigger mechanism in all cases where the sensor means does not detect skin.

It can also be advantageous to arrange cooling means at the front end of the device. When the flash bulb is ignited and light is emitted heat is generated. This heat is generated both from the ignition of the flash bulb device and by the penetration of the light through the skin layer. By arranging cooling means at the front end of the device this heat is cooled such that the patient will not feel the heat sensation and burning sensation which can otherwise be accompanied by this sort of treatment.

Using the device according to the invention, a method of treatment with light energy especially treatment of skin disorders such as unwanted hair removal, discolouring, smoothening of wrinkles is provided.

The method comprises the steps of providing a pulsed light output from a non-laser device. An incoherent light source is used which light is directed in the shape of light pulses to a skin area to be treated. By focusing the light by means of a reflector and filtering the light through a prism or iris, and optionally by coating the sides of the prism the wavelength of the emitted light can be controlled to be in the range between 550 and 1050 nm, and furthermore selecting the ignitable materials in the incoherent light source such that the emitted light energy is controlled in a very safe and side effect free method of therapeutic treatment a user-friendly method is achieved.

By selecting and controlling the emitted light within the wavelengths of between 550 to 1050 nm important safety aspects are achieved. With wavelengths above 1050 nm water will absorb a large part of the emitted energy which can cause the water to boil. When the water is present in the skin to be treated this can have some serious side effects and disadvantages to the treatment. On the other hand when filtering wavelengths below 550 nm away, it is achieved that less energy is absorbed in the blood stream. If too much energy is absorbed in the blood stream the blood will expand and burst some of the tiny vessels in the skin. This will give rise to discoloration and maybe pain for the patient in treatment. It is therefore desirable to keep the emitted light within wavelengths of 550 nm to 1050 nm.

In the description above, the light source device has been described as containing ignitable materials. These ignitable materials can in the layered versions be separated by delaying fuse layers which will delay the ignition of the following layer. Alternatively, mesh arrangements were described. Another possibility is to have ignitable gases in the light bulb device. The different types of gas can be separated by thin membranes so that when one gas is ignited it burns itself and the membrane and thereby ignites the following gas containing space. This type of light source is however less attractive to use in that the gases usually by ignition creates a violent explosion when emitting the light. The rather thin walled light source devices can rarely cope with the interior pressure from gas explosions and will burn and fragment causing tiny fragments of glass to be present in the vicinity of the treatment area. This is not a desirable situation.

Tests have indicated that prior to treatment it can be advantageous to treat the skin in the treatment area with a so called ultrasound gel. Test indicate that such a treatment allows up to about 20% more light to penetrate the skin layers. This in turn makes the treatment more efficient at lower energy levels.

### Description of the drawing

The invention will now be explained with reference to the accompanying drawing, wherein
- fig. 1: illustrates a self-contained device according to the invention;
- fig. 2: illustrates a light source having more ignition stages and
- fig. 2b: illustrates another light source having multiple ignition stages and
- fig. 2c: illustrates another embodiment of a light source having multiple ignition stages and
- fig. 4: illustrates in schematic form a construction of the device according to the invention and
- fig. 5: illustrates the distance keeping properties of the prism device.

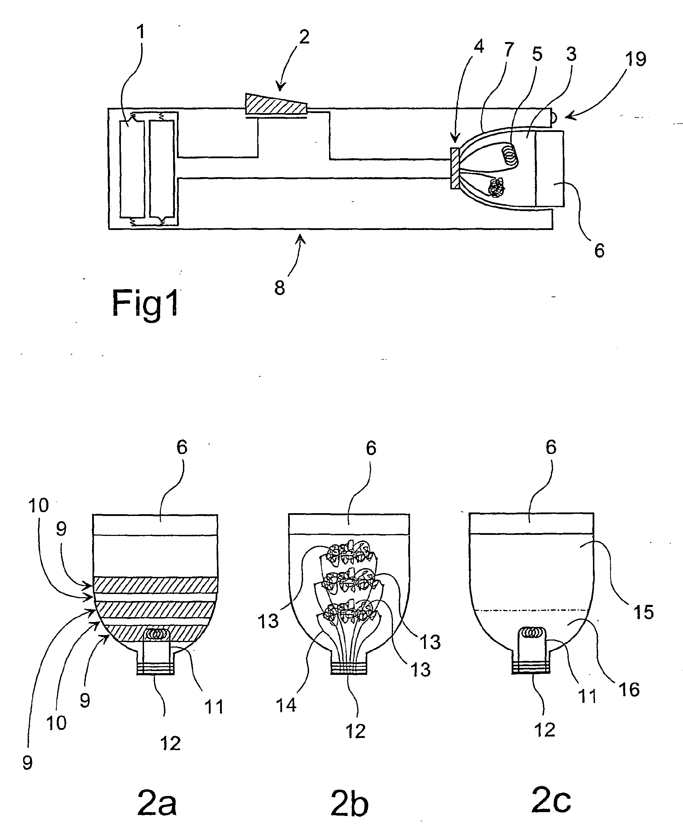

In figure 1 is illustrated a self-contained version of the device according to the invention. The device can in some aspects be compared to a common flash light. Batteries 1 represent the energy source in this version and the trigger mechanism 2 is mounted as a switch on a wire guiding current from the batteries to a disposable incoherent light source 3. The disposable light source is in this embodiment in the shape of a disposable flash bulb, which is mounted in a socket 4. The system can be compared to the old-fashioned way of performing flash photography used before electronic flash devices became widely available. Before each use a new suitable flash bulb, that is to say an incoherent light emitting device 3 is mounted in the socket 4. By making the electrical contact by the triggering device 2 current is led to the flash bulb 3, whereby the ignitable material 5 is ignited and emits light. In this embodiment of the invention a light source having a prism or iris 6 mounted in front of the ignitable material is illustrated. Part of the emitted light arising from the ignition of the light emitting material 5 passes straight through the prism 6 and into the treatment area. The rest of the light is bounced off the reflector 7 and guided in a comparable manner to a head light on a car out through the prism 6 and into the treatment area. By this arrangement substantially all the light emitted by ignition of ignitable material 5 will be guided towards the treatment area on a patient. This allows for the relatively low energy levels used in the system, which makes it safer for the patient to use.

In these examples the prism or iris is an integral part of the disposable light device. The prism can however be a part of the device, which makes the disposable light source a simpler, and thereby cheaper part.

All the above mentioned components of the device are in this embodiment of the invention arranged in a housing 8. The housing can be made from any suitable material for example metals or plastics.

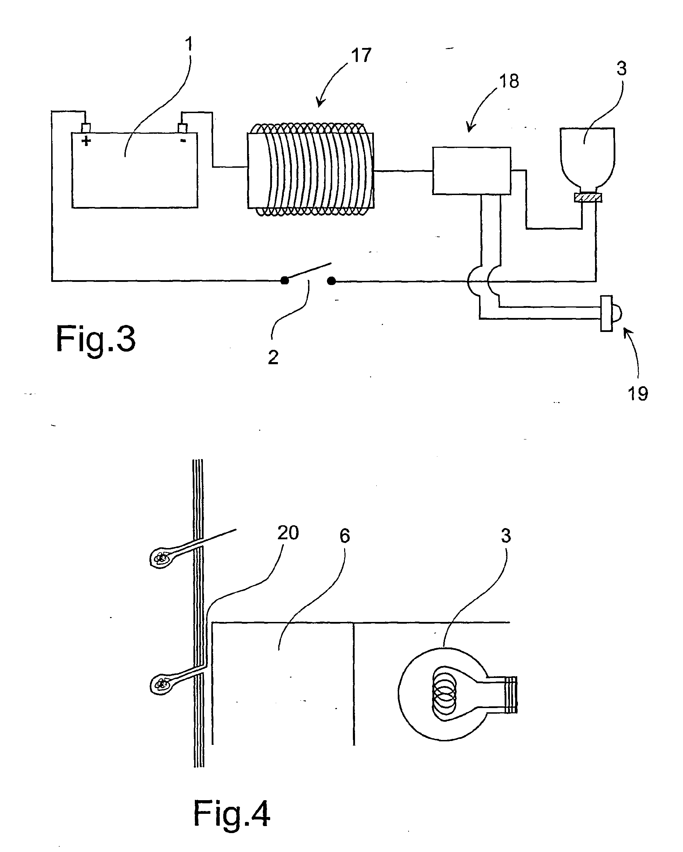

In figures 2a, 2b and 2c three different embodiments of a suitable incoherent light source are illustrated.

In figure 2a a light source, wherein a layered structure of ignitable materials 9, separated by delaying fuses 10 is illustrated. Furthermore, the ignition device 11 is seen placed in the lowermost layer of ignitable material. The lowermost part of the light source is equipped with a footing 12, which is adapted to be mounted in the socket 4 of the device illustrated in figure 1. In front of the ignitable materials seen in the direction of mounting the light source in the device in figure 1 a prism or iris 6 is arranged.

In figure 2b a similar device having a footing 12 and prism or iris is illustrated. Instead of layered ignitable materials the ignitable material in the embodiment shown in figure 2b is in the shape of metal meshes 13 with separate electrodes 14, whereby a circuitry arranged in the device shown in figure 1 will control the ignition of the separate meshes according to a pre-scheduled light pulse programme.

The light source illustrated in figure 2c consists of the footing 12 and a prism/iris 6 and a ignition device 11. The interior of the light source is in this embodiment illustrated as having two separate chambers 15, 16. It is envisaged that two different gasses can be arranged in the chambers 15, 16, whereby once the first gas in chamber 16 is ignited an initial flash will appear which will also ignite the gas arranged in chamber 15. In this manner a two stage light pulse will be achieved.

In figure 3 a schematic presentation of the device shown in figure 1 is illustrated. The energy source 1 is connected to transformation means 17, which controls the current in the circuitry. Optionally, a circuitry controlling the current pulse is sent to the light source 3 can be arranged. This is particularly important in the embodiments, where a multi-stage lighting device is used, or where it is desirable to have sensing means arranged in order to determine the skin characteristics prior to treatment. In this embodiment the circuitry 18 is illustrated as being connected both to the light source device 3 and a sensing means 19.

The sensing means 19 determines the skin characteristics on which the light treatment can be adjusted. Furthermore, the sensing means 19 is a safety measure in that the sensor can sense, whether or not the light source 3 is placed in front of a skin area to be treated or by mistake is placed in front of an eye or other organ, which can be damaged by the intense light treatment. In this instance sensor means make it impossible to progress with the treatment, when the trigger means 2 are activated. It can therefore be regarded as a fail/safety mechanism which further improves the usability of the device.

In figure 4 a device brought into the treatment situation is illustrated. The light source 3 is illustrated as a traditional single stage light bulb, but can be any of the other light sources described above. The prism 6 arranged in front of the light source also has the function of distance keeping. By giving the prism/iris a certain thickness a minimum distance will at all times be kept between the lights source and the surface of the skin to be treated. It is hereby assured that the correct level of light energy is transmitted on to the treatment surface, whereby burns or other damage arising from too intense a treatment can be avoided. By arranging the prism/iris as a plurality of separate lenses, the light will be evenly distributed to the surface 20 of the prism, whereby a larger surface can be treated with a even light energy.

## Claims

1. Therapeutic treatment device especially for treatment of skin disorders such as unwanted hair, discolouring, smoothing wrinkles, said device comprising a disposable incoherent light source (3), an energy source (1) and a trigger device (2), said disposable incoherent light source (3) comprising one or more ignitable materials (5) or gasses, **characterised in that** said incoherent light source (3) is a single use incoherent light source wherein the ignitable material (5) or gas is ignited during use and thus said incoherent light source (3) expires after said single use, such that said incoherent light source needs to be replaced before a subsequent use of said device.

2. Device according to claim 1, **characterised in that** the ignitable materials are arranged in layers (9), each layer is separated by a delaying fuse (10).

3. Device according to claim 1, **characterised in that** the light source (3) comprises one or more ignitable metal meshes (13), each mesh being connected via an ignition control device to the energy source (1) by separate electrodes for each mesh (13).

4. Device according to claim 3, wherein the one or more meshes (13) are arranged in an atmosphere of oxygen or xenon.

5. Device according to any of claims 1 to 4, **characterised in that** a prism (6) or an iris is arranged between the light source (3) and the object to be treated, said prism collecting and guiding the emitted light to the surface to be treated.

6. Device according to claim 5, **characterised in that** the prism (6) consists of a plurality of separate prisms arranged to collect and guide the emitted light.

7. Device according to claim 5 or 6, **characterised in that** the prism (6) functions as distance keeping means, whereby the distance between the light source (3) and the surface to be treated is held constant.

8. Device according to claims 5 to 7, **characterised in that** the sides of the prism (6) not adapted to contact the skin or the light source (3) are coated with laser dye.

## Patentansprüche

1. Therapeutische Behandlungsvorrichtung, Insbesondere zur Behandlung von Hautstörungen wie unerwünschten Haaren, Verfärbungen, Glätten von Falten, wobei die Vorrichtung eine einmal verwendbare inkohärente Lichtquelle (3), eine Energiequelle (1) und eine Auslösevorrichtung (2) umfasst, wobei die einmal verwendbare inkohärente Lichtquelle (3) ein oder mehrere entzündbare Materiallen (5) oder Gase enthält, **dadurch gekennzeichnet, dass** die inkohärente Lichtquelle (3) eine inkohärende Einweg-Lichtquelle ist, In welcher das entzündbare Material (5) oder Gas während des Gebrauchs entzündet wird, wodurch die inkohärente Lichtquelle (3) nach dem einmaligem Gebrauch erlischt, so dass diese inkohärente Lichtquelle ersetzt werden muss, bevor die Vorrichtung nochmals Verwendung findet.

2. Vorrichtung nach Anspruch 1, **dadurch gekennzeichnet, dass** die entzündbaren Materiallen in Schichten (9) angeordnet sind, wobei die Schichten jeweils durch Verzögerungszünder (10) voneinander getrennt sind.

3. Vorrichtung nach Anspruch 1, **dadurch gekennzeichnet, dass** die Lichtquelle (3) ein oder mehrere zündbare Metall-Maschengitter (13) umfasst, wobei alle Gitter über eine Zündsteuervorrichtung mittels separater Elektroden für jedes Gitter (13) mit der Energiequelle (1) verbunden sind.

4. Vorrichtung nach Anspruch 3, wobei das eine oder mehrere Gitter (13) in einer Sauerstoff- oder Xenon-Atmosphäre angeordnet sind.

5. Vorrichtung nach einem der Ansprüche 1 bis 4, **dadurch gekennzeichnet, dass** ein Prisma (6) oder eine Irisblende zwischen der Lichtquelle (3) und dem zu behandelnden Objekt angeordnet ist, wobei das Prisma das emittierte Licht sammelt und auf die zu behandelnde Oberfläche leitet.

6. Vorrichtung nach Anspruch 5, **dadurch gekennzeichnet, dass** das Prisma (6) aus einer Vielzahl von separaten Prismen besteht, welche so angeordnet sind, dass sie das emittierten Licht sammeln und leiten.

7. Vorrichtung nach Anspruch 5 oder 6, **dadurch gekennzeichnet, dass** das Prisma (6) als Abstandshalter fungiert, wobei der Abstand zwischen der Lichtquelle (3) und der zu behandelnden Oberfläche konstant gehalten wird.

8. Vorrichtung nach Anspruch 5 bis 7, **dadurch gekennzeichnet, dass** die Selten des Prismas (6), welche nicht dafür vorgesehen sind, mit der Haut oder der Lichtquelle (3) in Kontakt zu treten, mit Laserfarbe beschichtet sind.

## Revendications

1. Dispositif de traitement thérapeutique pour traiter en particulier les affections cutanées telles que les poils indésirables, les décolorations, et l'atténuation des rides, ledit dispositif comprenant une source de lumière incohérente (3) à usage unique, une source d'énergie (1) et un dispositif de déclenchement (2), ladite source de lumière incohérente (3) à usage unique comprenant un ou plusieurs matériaux (5) ou gaz inflammables, **caractérisé en ce que** ladite source de lumière incohérente (3) est une source de lumière incohérente à usage unique dans laquelle le matériau (5) ou gaz inflammable est enflammé pendant l'usage et, par conséquent, ladite source de lumière incohérente (3) s'éteint après ledit usage unique, de sorte que ladite source de lumière incohérente doit être remplacée avant un usage ultérieur dudit dispositif.

2. Dispositif selon la revendication 1, **caractérisé en ce que** les matériaux inflammables sont disposés en couches (9), chaque couche étant séparée par un fusible temporisé (10).

3. Dispositif selon la revendication 1, **caractérisé en ce que** la source de lumière (3) comprend une ou plusieurs mailles (13) métalliques inflammables, chaque maille étant reliée via un dispositif de commande d'inflammation à la source d'énergie (1) par des électrodes séparées pour chaque maille (13).

4. Dispositif selon la revendication 3, dans lequel les une ou plusieurs mailles (13) sont disposées dans une atmosphère d'oxygène ou de xénon.

5. Dispositif selon l'une quelconque des revendications 1 à 4, **caractérisé en ce qu'**un prisme (6) ou un diaphragme est disposé entre la source de lumière (3) et l'objet à traiter, ledit prisme recueillant et guidant la lumière émise jusqu'à la surface à traiter.

6. Dispositif selon la revendication 5, **caractérisé en ce que** le prisme (6) consiste en une pluralité de prismes séparés disposés pour recueillir et guider la lumière émise.

7. Dispositif selon la revendication 5 ou 6, **caractérisé en ce que** le prisme (6) fonctionne en tant que moyen de maintien de distance, la distance entre la source de lumière (3) et la surface à traiter étant maintenue constante.

8. Dispositif selon les revendications 5 à 7, **caractérisé en ce que** les côtés du prisme (6) non adaptés pour être en contact avec la peau ou la source de lumière (3) sont revêtus d'un colorant laser.
